# EUROPÄISCHE PATENTSCHRIFT

(11) **EP 3 863 567 B1**
(45) Veröffentlichungstag und Bekanntmachung des Hinweises auf die Patenterteilung: **03.04.2024**
(21) Anmeldenummer: 19719438.4
(22) Anmeldetag: 05.04.2019
(51) Int. Cl.: A61F 2/24, A61F 2/00

(54) **IMPLANTAT ZUR BEHANDLUNG UND/ODER ZUM ERSATZ EINER ENTZÜNDETEN, THROMBOSIERTEN ODER DEGENERIERTEN HERZKLAPPE**
IMPLANT FOR TREATING AND/OR FOR REPLACING AN INFLAMED, THROMBOSED OR DEGENERATED HEART VALVE
IMPLANT POUR LE TRAITEMENT ET/OU POUR LE REMPLACEMENT D'UNE VALVE CARDIAQUE TOUCHÉE PAR UNE INFLAMMATION, UNE THROMBOSE OU UNE DÉGÉNÉRESCENCE

(30) Priorität: 10.10.2018 US 201862743828 P
(43) Veröffentlichungstag der Anmeldung: 18.08.2021
(73) Patentinhaber: Devie Medical GmbH, 07743 Jena (DE)
(72) Erfinder: FIGULLA, Hans Reiner, 07749 Jena (DE); SEIDEL, Raphael A., 07749 Jena (DE)
(74) Vertreter: Meissner Bolte Partnerschaft mbB
(86) Internationale Anmeldenummer: PCT/EP2019/058666
(87) Internationale Veröffentlichungsnummer: WO 2020/074130

(56) Entgegenhaltungen:
- US-A1- 2013 035 759
- US-A1- 2016 228 248
- US-A1- 2018 000 586
- US-A1- 2018 206 982
- US-A1- 2018 250 126

## Beschreibung

Die vorliegende Erfindung ist aus dem Gebiet der Herzchirurgie und Kardiologie und betrifft insbesondere die Behandlung und/oder den Ersatz einer an einer Entzündung und/oder Infektion erkrankten Herzklappe.

Die Druckschrift US 2018/250126 A1 betrifft eine expandierbare Ersatzherzklappe, welche in einem komprimierten Zustand insbesondere minimal-invasiv in den Körper eines Patienten einführbar und im Implantationsort an einer insuffizienten Herzklappe derart expandierbar ist, dass die Ersatzherzklappe die Funktion einer nativen Herzklappe übernimmt. Die Ersatzherzklappe ist ferner geeignet, eine insuffiziente Mitralklappe zu behandeln. Zu diesem Zweck wird im Bereich der insuffizienten Mitralklappe eine Verankerungsstruktur eingesetzt, wobei in dieser Verankerungsstruktur dann ein Stent mit der Ersatzherzklappe positioniert werden kann.

Es gibt vier Klappen in den nativen Herzen, die zum Ausrichten des Blutflusses durch die beiden Seiten des Herzens in einer Richtung nach vorne dienen. An der linken (systemischen) Seite des Herzes sind die Mitralklappe, die zwischen dem linken Vorhof und der linken Kammer liegt, und die Aortenklappe, die zwischen der linken Kammer und der Aorta liegt, angeordnet. Diese beiden Klappen leiten sauerstoffreiches Blut, welches von der Lunge kommt, durch die linke Seite des Herzens hindurch in die Aorta hinein zum Verteilen auf den Körper. An der rechten (pulmonalen) Seite des Herzens sind die Trikuspidalklappe, die zwischen dem rechten Vorhof und der rechten Kammer liegt, und die Pulmonalklappe, die zwischen der rechten Kammer und der Pulmonalarterie liegt. Diese beiden Klappen leiten sauerstoffarmes Blut, welches von dem Körper kommt, durch die rechte Seite des Herzens hindurch in die Pulmonalarterie hinein zum Verteilen auf die Lunge, wo es wieder sauerstoffangereichert wird, um den Kreislauf von neuem zu beginnen.

Alle vier dieser Herzklappen sind passive Strukturen dadurch, dass sie selbst keinerlei Energie aufwenden und keinerlei aktive kontraktile Funktion durchführen. Sie bestehen aus beweglichen "Segeln", die gelegentlich auch als "Leaflets" bezeichnet werden, und die dafür bestimmt sind, in Erwiderung auf unterschiedliche Drücke an jeder Seite der Klappe einfach zu öffnen und zu schließen. Die Mitralund Trikuspidalklappe werden wegen ihrer Lage zwischen einem Vorhof und einer Kammer an jeder Seite des Herzens als "Vorhof-Kammerklappen" bezeichnet. Die Mitralklappe hat zwei Segel und die Trikuspidalklappe hat drei Segel. Die Aortenund Pulmonalklappe werden als "halbmondförmige Klappen" bezeichnet, welche passenderweise "Segel" genannt werden. Die Aorten- und Pulmonalklappen haben jeweils drei Segel.

Die Endokarditis ist eine Entzündung der Herzinnenhaut (Endokard), welche die Herzhöhlen und den herznahen Anteil der Arterien und Venen auskleidet und auch die Struktur der Herzklappensegel bildet. Zahlreiche Mikroorganismen können eine Endokarditis verursachen - besonders grampositive Bakterienspezies wie Streptokokken, Enterokokken und Staphylokokken. Wenn diese im Zuge einer Bakteriämie das Endokard besiedeln, entsteht eine infektiöse Endokarditis.

Abhängig von der Art des Auslösers tritt eine mortale Folge der Endokarditis in ca. 25% der Fälle ein.

Eine Möglichkeit zur Behandlung einer Endokarditis ist nach dem Stand der Technik die chirurgische Entfernung der entzündeten Herzklappe und die Implantation einer künstlichen Herzklappe bzw. Ersatzherzklappe. In der Regel werden hierfür arbeits- und kostenintensive Operationen durchgeführt, die mit einer hohen Patientenbelastung und einem beträchtlichen Risiko verbunden sind. Im Detail wird der Brustkorb des Patienten eröffnet, das Herz mittels kardiopleger Lösung zum Stillstand gebracht, die körpereigene Herzklappe entfernt und an dessen Stelle eine künstliche Herzklappe an das körpereigene Gewebe angenäht. Neuere Methoden, wie in WO2006/076890 A1 dargestellt, sehen eine Transkatheter-Implantation künstlicher Herzklappen unter Einsatz eines Stents als Stützstruktur vor, die aber nur bei degenerierten Aortenklappenerkrankungen einsetzbar sind.

Bei entzündeten Herzklappen mit bakteriellen An- bzw. Ablagerungen im Sinne von größeren Vegetationen sind bisher nur operative Verfahren anwendbar, die das infizierte, entzündete Material im umliegenden Gewebe der Herzklappe und die Herzklappe selbst entfernen und eine künstliche Herzklappe einnähen. Die kathetergeführten Klappenimplantationen sind bei entzündeten Herzklappen kontraindiziert, da, wie oben genannt, dass entzündete Material hierbei nicht entfernt werden kann. Gerade bei der Endokarditis sind aber die operativen Verfahren, die bei 75% der Fälle mit Endokarditis angewandt werden (25% werden nicht operiert, weil die Patienten entweder nicht mehr operabel sind oder weil es sich nur um eine leichte Entzündung handelt) aber besonders risikoreich. Dies liegt daran, dass die Patienten mit einer Endokarditis meist erhebliche Begleiterkrankungen aufweisen, die im Zusammenhang mit der Herzklappenentzündung stehen, wie Embolisationen in das Gehirn, Nieren, Haut und weitere Organe und weil die Patienten septisch, d.h. mit hohem Fieber und entzündungsbedingten Beeinträchtigungen und einer destruierten Klappe, die zu einer Herzinsuffizienz geführt hat, notfallmäßig operiert werden.

Auf der Grundlage dieser Problemstellung liegt der Erfindung somit die Aufgabe zu Grunde, durch die Implantation einer kathetergeführten Herzklappe, die lokal gleichzeitig die Infektion und/oder Thrombose und die degenerierte Herzklappe behandeln kann.

Im Hinblick auf das Implantat wird die der Erfindung zu Grunde liegende Aufgabe insbesondere durch den Gegenstand des unabhängigen Patentanspruches 1 gelöst, wobei vorteilhafte Weiterbildungen des erfindungsgemäßen Implantates in den entsprechenden Unteransprüchen angegeben sind.

Demgemäß betrifft die Erfindung insbesondere ein Implantat zur Behandlung oder zum Ersatz einer an einer Entzündung und/oder Infektion erkrankten Herzklappe, wobei das Implantat eine Fangeinrichtung aufweist, welche in einem komprimierten Zustand minimal-invasiv in den Körper des Patienten einführbar und im Implantationsort an der erkrankten Herzklappe expandierbar ist. Die Fangeinrichtung ist insbesondere ausgebildet, zumindest im implantierten und expandierten Zustand Gewebeauflagerung insbesondere in Gestalt einer Herzklappenvegetation zu kompartimentieren, und zwar insbesondere indem die Gewebeauflagerung zumindest bereichsweise von der Fangeinrichtung umgriffen und/oder aufgenommen wird.

Für die Fangeinrichtung des erfindungsgemäßen Implantates kommen verschiedene Ausführungsformen in Frage. So ist es beispielsweise denkbar, dass die Fangeinrichtung eine netz- oder gitter- oder membranartige Struktur aufweist, über welche die Gewebeauflagerung an der erkrankten nativen Herzklappe zumindest bereichsweise umgriffen und/oder aufgenommen werden kann, um diese so entsprechend zu kompartimentieren.

Alternativ oder zusätzlich hierzu ist es aber auch denkbar, dass die Fangeinrichtung zumindest bereichsweise eine Gewebestruktur insbesondere aus einem bioresorbierbaren Material aufweist, um so im implantierten und expandierten Zustand des Implantates bzw. der Fangeinrichtung eine Einkapselung der Gewebeauflagerung im Bereich der zu behandelnden nativen Herzklappe ermöglichen zu können. Die Gewebestruktur der Fangeinrichtung kann beispielsweise aus bioresorbierbaren Fäden, insbesondere Mikro-Fasern, aufgebaut sein, die beispielsweise derart untereinander vernetzt sind, dass ein Vlies oder vliesartige Struktur gebildet wird.

Selbstverständlich kommen aber auch andere Ausführungsformen sowie deren Kombination für die Fangeinrichtung in Frage.

Um eine möglichst genaue und sichere Fixierung und/oder Positionierung der Fangeinrichtung im Implantationsort ermöglichen zu können, ist der Fangeinrichtung erfindungsgemäß eine geeignete Verankerungsstruktur zugeordnet. Dabei ist vorgesehen, dass eine Fangstruktur geeignet mit der Verankerungsstruktur verbunden ist, um so insgesamt die Fangeinrichtung auszubilden. In Blutstromrichtung gesehen sollte gemäß Ausführungsformen der Fangeinrichtung die mit der Verankerungsstruktur verbundene Fangstruktur sicher stromabwärts in ein strömungsmäßig mit der erkrankten Herzklappe verbundenes Gefäß erstrecken, wenn das Implantat im implantierten Zustand vorliegt.

Darüber hinaus weist die Fangstruktur im expandierten Zustand mindestens einen Aufspannbereich auf, welcher ausgebildet ist, im expandierten und implantierten Zustand der Fangeinrichtung mit mindestens einem Herzklappensegel der erkrankten Herzklappe derart wechselzuwirken, dass das mindestens eine Herzklappensegel in Richtung einer Gefäßwand gedrückt wird. Vorzugsweise ist der Aufspannbereich der Fangeinrichtung ausgebildet, dass möglichst alle Herzklappensegel der erkrankten Herzklappe geeignet in Richtung der Gefäßwand gedrückt werden. Auf diese Weise können nicht nur die Gewebeauflagerung (insbesondere Herzklappenvegetation) an den Segeln der erkrankten Herzklappe kompartimentiert werden, sondern es wird gleichzeitig auch schon die erkrankte Herzklappe für den Ersatz durch eine Ersatzherzklappe (Herzklappenprothese) vorbereitet.

Als Aufspannbereich, über den der bzw. die Herzklappensegel der erkrankten nativen Herzklappe in Richtung der Gefäßwand gedrückt werden, kann auf unterschiedliche Weise ausgebildet sein. Insbesondere bietet es sich an, dass der mindestens eine Aufspannbereich zu mindestens einen Aufspannarm oder Aufspannbügel aufweist, welcher ausgebildet ist, bei der Expansion der Fangeinrichtung im Implantationsort zumindest bereichsweise in radialer Richtung zu expandieren.

Um eine möglichst wirksame Kompartimentierung der Gewebeauflagerung erzielen zu können, ist gemäß Ausführungen der Erfindung vorgesehen, dass der Aufspannbereich an einem der Verankerungsstruktur abgewandten, distalen Endbereich mindestens einen zumindest im Wesentlichen in Richtung der Verankerungsstruktur zeigenden Bereich aufweist.

Gemäß Ausführungsformen des erfindungsgemäßen Implantats weist dieses ferner eine insbesondere separat von der Fangeinrichtung implantierbare Struktur auf, welche insbesondere einen im Wesentlichen ringförmigen Aufbau aufweist und in die Taschen (zwischen den Segeln und der Gefäßwand) der nativen Herzklappe einsetzbar ist und als Widerlager insbesondere für die Fangeinrichtung und/oder den Aufspannbereich des Implantats dient.

Beispielsweise kann diese insbesondere separat von der Fangeinrichtung implantierbare Struktur als ein insbesondere zumindest im Wesentlichen ringförmiges Widerlager-Implantat ausgeführt sein, welches derart im Implantationsort implantierbar ist, dass im implantierten Zustand des Implantates zwischen dem Implantat und dem Widerlager-Implantat zumindest bereichsweise mindestens ein Klappensegel der zu behandelnden Herzklappe aufnehmbar ist.

Gemäß einem weiteren Aspekt der vorliegenden Erfindung dient das Implantat insbesondere zum Ersatz einer an einer Entzündung und/oder Thrombose und/oder Degeneration erkrankten, und insbesondere einer an einer Endokarditis erkrankten Herzklappe, wobei das Implantat zusätzlich zu der bereits diskutierten Fangeinrichtung eine expandierbare Ersatzherzklappe bzw. Herzklappenprothese aufweist, welche in einem komprimierten Zustand insbesondere minimal-invasiv in den Körper des Patienten einführbar und im Implantationsort an der erkrankten Herzklappe derart expandierbar ist, dass die Ersatzherzklappe bzw. Herzklappenprothese zumindest im Wesentlichen die Funktion einer nativen Herzklappe übernimmt.

Gemäß Ausführungsformen ist der Ersatzherzklappe mindestens ein geeigneter Stent zugeordnet, um die Ersatzherzklappe insbesondere im implantierten Zustand geeignet abzustützen und zu halten. Der Stent stellt somit die Träger- und Stützstruktur der Ersatzherzklappe dar und dient gleichzeitig der Positionierung und Fixierung der Ersatzherzklappe am Implantationsort.

Selbstverständlich ist es in diesem Zusammenhang aber auch denkbar, dass der Ersatzherzklappe nicht nur ein einziger Stent, sondern mehrere, insbesondere zwei oder drei Stents zugeordnet sind, um beispielsweise im Implantationsort eine so genannte Stent-in-Stent-Lösung realisieren zu können, wonach ein erster Stent im Bereich zwischen Aortenwand und der nativen Herzklappensegel als Widerlager fixiert ist, und ein zweiter Stent mit der daran befestigten Ersatzherzklappe in den bereits implantierten Stent eingesetzt wird. Eine solche Stent-In-Stent-Lösung bietet den Vorteil, dass die Ersatzherzklappe einen festen Sitz hat und biologisches Material effizienter kompartimentiert werden kann. Ein weiterer Vorteil ist darin zu sehen, dass das Implantat zum Ersatz einer an einer Endokarditis erkrankten Herzklappe komponentenweise implantiert werden kann, so dass ein Einführ-Kathetersystem mit einem maximalen Katheterdurchmesser von insbesondere weniger als 22 Fr verwendet werden kann, da beide Komponenten auch Träger von Wirksubstanzen seien können und somit bei gleichem Katheterdurchmesser insgesamt ein größeres Volumen eingeführt werden kann.

Gemäß Realisierungen des erfindungsgemäßen Implantates ist vorgesehen, dass diese zusätzlich zu der Fangeinrichtung zur Kompartimentierung der Gewebeauflagerungen eine geeignete Ersatzherzklappe mit einem entsprechend zugeordneten Stent aufweist, wobei dieser Stent die Träger- und Stützstruktur der Ersatzherzklappe darstellt. Darüber hinaus kann der der Ersatzherzklappe zugeordnete Stent ausgebildet sein, im Implantationsort die Herzklappensegel der nativen Herzklappe zu verdrängen, insbesondere in radialer Richtung und vorzugsweise derart, dass die Herzklappensegel der nativen Herzklappe an oder in Richtung der Gefäßwand gedrückt werden.

In diesem Zusammenhang bietet es sich auch an, dass der der Ersatzherzklappe zugeordnete Stent, gleichzeitig eine oder die Verankerungsstruktur der Fangeinrichtung ausbildet. Mit anderen Worten, dem der Ersatzherzklappe zugeordnete Stent kann eine Doppelfunktion zukommen: zum einen kann der Stent als Trägerund Stützstruktur der Ersatzherzklappe dienen, wobei gleichzeitig die Positionierung und Fixierung der Ersatzherzklappe im Implantationsort an der erkrankten nativen Herzklappe sichergestellt ist. Andererseits kann der, der Ersatzherzklappe zugeordnete Stent, als Verankerungsstruktur für die Fangeinrichtung dienen, wobei dann eine entsprechende Fangstruktur an dem als Verankerungsstruktur der Fangeinrichtung dienenden Stent befestigt oder befestigbar ist.

Um zu erreichen, dass das Implantat in einer möglichst minimal-invasiven Weise unter Verwendung eines möglichst dünnen Einführ-Kathetersystems in den Körper des Patienten einführbar ist, bietet es sich an, dass das Implantat ausgebildet ist, im Implantationsort an der erkrankten nativen Herzklappe zeitlich gesehen nacheinander schrittweise zu expandieren. Gemäß Ausführungsformen ist dabei vorgesehen, dass in einem ersten Schritt die Fangeinrichtung expandiert, um die Gewebeauflagerungen zu einzufangen und zu kompartimentieren, wobei anschließend eine Expansion der Ersatzherzklappe erfolgt. Selbstverständlich ist die Erfindung jedoch nicht auf diese Reihenfolge beschränkt. Vielmehr kann das Implantat auch ausgebildet sein, dass in einem ersten Schritt die Ersatzherzklappe expandiert und dadurch die Herzklappensegel der nativen Herzklappe mit den Gewebeauflagerungen radial nach außen drückt, wobei anschließend mit Hilfe der Fangeinrichtung die radial nach außen gedrückten Herzklappensegel und insbesondere Gewebeauflagerungen an den Herzklappensegeln der nativen Herzklappe - beispielsweise auch in Verbindung mit einer zuvor separat eingefügten Widerlager-Struktur - geeignet kompartimentiert werden.

In einer bevorzugten Weiterbildung ist das erfindungsgemäße Implantat ferner ausgebildet, im implantierten Zustand Wirkstoffe freizusetzen. Auf diese Weise kann vor Ort, das heißt in situ und topisch, die Entzündung, Thrombose oder Degeneration der nativen Herzklappe behandelt und die Infektion, Thrombose oder Degeneration kontrolliert werden, ohne das eine zusätzliche Belastung/Gefährdung des Patienten durch eine chirurgische Behandlung der nativen Herzklappe erforderlich ist.

Als Wirkstoffe kommen insbesondere antimikrobielle, antibiotische, bakterizide, antithrombotische, thrombolytische, zelltoxische und/oder vergleichbare Wirkstoffe in Frage, die mit Hilfe des erfindungsgemäßen Implantates am Ort der Erkrankung an das umliegende Gewebe und in die Blutbahn abgebbar sind.

Beispielsweise ist es in diesem Zusammenhang denkbar, dass zum Freisetzen der Wirkstoffe die Fangeinrichtung und/oder eine gegebenenfalls zum Implantat gehörende Ersatzherzklappe bzw. ein einer solcher Ersatzherzklappe zugeordneter Stent eine Beschichtung mit den Wirkstoffen aufweist.

Gemäß Ausführungsformen der vorliegenden Erfindung kommt zum Freisetzen der Wirkstoffe eine bioresorbierbare Struktur zum Einsatz, welche Teil des erfindungsgemäßen Implantates ist, und welche insbesondere eine Faden- und/oder Vliesstruktur und/oder Membranstruktur mit einem Wirkstoff aufweist.

Das erfindungsgemäße Implantat ist insbesondere ausgelegt, die oben genannten Wirkstoffe am Ort der Erkrankung an das umliegende Gewebe und in die Blutbahn abzugeben.

Dazu kann das erfindungsgemäße Implantat ein Stent-Wirkstoff-System mit mindestens einem Stent aufweisen, an dem die Fangeinrichtung und/oder eine Ersatzherzklappe befestigt sind/ist. Insbesondere weist das erfindungsgemäße Implantat biokompatible Materialien auf, um eine gute Integrierbarkeit des Systems in das biologische Umfeld nach der Implantation zu gewährleisten. Der mindestens eine Stent stellt die Träger- und Stützstruktur der Ersatzherzklappe dar und dient gleichzeitig der Positionierung und Fixierung des erfindungsgemäßen Systems und insbesondere der Fangeinrichtung am Implantationsort.

Das Stent-Wirkstoff-System muss die insuffiziente, native Herzklappe radial verdrängen können, um die Herzvegetation mit der Fangeinrichtung zu kompartimentieren und die Ersatzherzklappe anstelle der nativen Herzklappe aufzuspannen und eine fehlerfreie Klappenfunktion während der Systole und Diastole des Herzens sicherzustellen. Auch muss der mindestens eine Stent dazu geeignet sein, während des periodischen Herzschlags der Ersatzherzklappe einen sicheren Halt zu bieten, so dass das erfindungsgemäße System nicht aufgrund wechselnder Druckverhältnissen im Herzen vom biologischen Gewebe, insbesondere von der Gefäßwand, abgelöst und vom Implantationsort ausgeschwemmt werden kann.

Hierzu kann das erfindungsgemäße System mindestens einen Stent auf, der per Ballonexpansion mit Hilfe eines Ballonkatheters expandiert und am Implantationsort positioniert werden kann. Dabei wird der komprimierte und im Katheter verborgene Stent durch einen mit Flüssigkeit oder Gas zu füllenden Katheterballon expandiert.

Alternativ kann der mindestens eine Stent des erfindungsgemäßen Systems ein selbstexpandierender Stent sein. Insbesondere besteht der Stent hierzu aus einer Formgedächtnislegierung, vorzugsweise aus Nitinol. Neben dem Formgedächtniseffekt bei einer spezifischen Sprungtemperatur, nahe der Körpertemperatur, weist Nitinol gleichfalls Superelastizität, Biokompatibilität und Korrosionsbeständigkeit auf. So wird Nitinol bereits vielfach in der Medizintechnik angewendet. Insbesondere die Superelastizität ist vorteilhaft hinsichtlich der komprimierten Zuführungsform eines Stents im Transkatheter-Verfahren und der Expansion am Implantationsort. Neben den zwei separat ausgeführten Expansionsverfahren ist gleichfalls auch eine Kombination beider Verfahren möglich. Insbesondere die radiale Vorspannkraft des Stents kann nach der Selbstexpansion durch eine Ballonexpansion noch zusätzlich gesteigert werden, wobei wiederrum eine höhere Stabilität des erfindungsgemäßen Systems im implantierten Zustand erreicht wird.

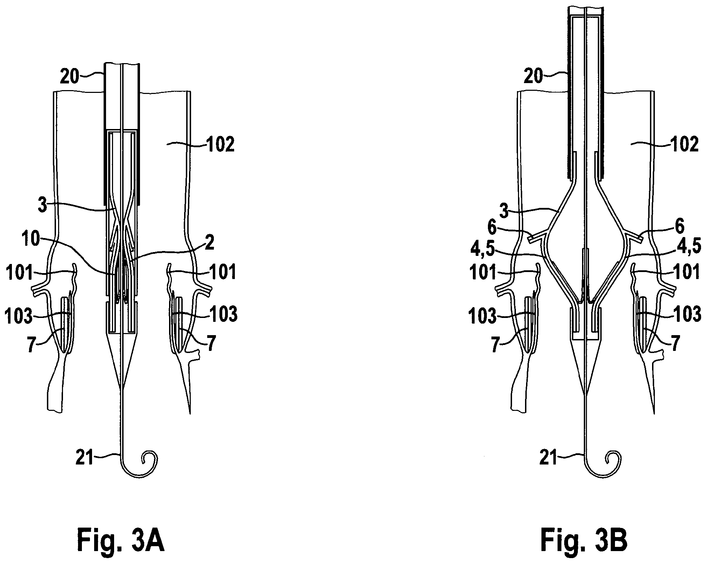

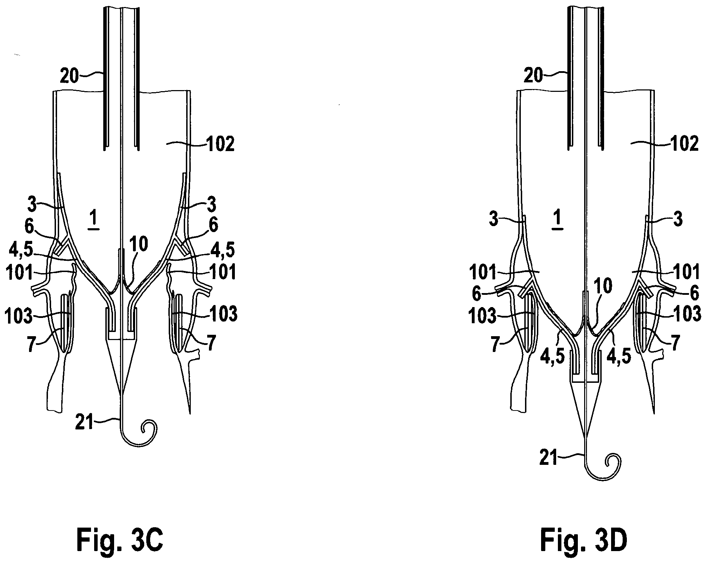

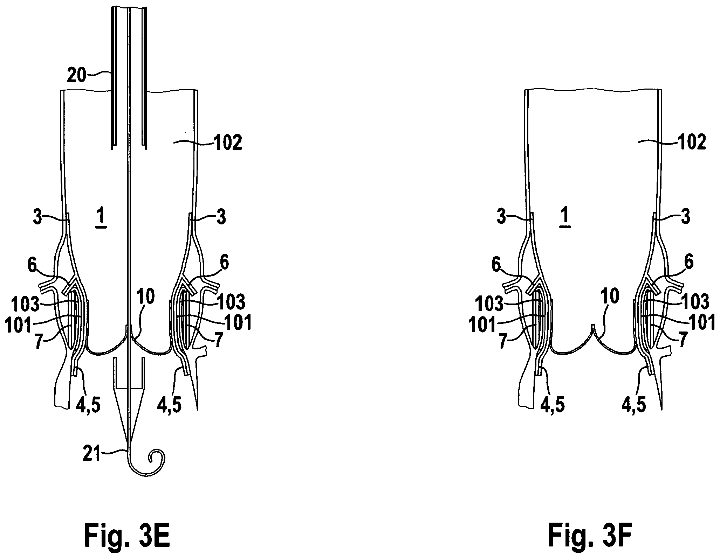

In diesem Zusammenhang ist es auch möglich, dass statt einer strebenartigen Stentstruktur mit Fangeinrichtung eine Gewebestruktur aus Nitinol-, einem anderen metallhaltigen Werkstoff, oder stabilen Polymerstreben als Gerüst zum Tragen der Ersatzherzklappe und Auffangrichtung dienen kann. Insbesondere eine gewebeartige Stentstruktur ist geeignet, eine Verkürzung, wie in FIG. 3A bis FIG. 3F dargestellt, der Fangeinrichtung auf die Ringstruktur des Auffanglagers zu erreichen.

Die an dem mindestens einen Stent befestigte Ersatzherzklappe kann eine Perikardklappe sein, eine biologische Herzklappe, eine künstliche Herzklappe, vorzugsweise bestehend aus biokompatiblen Materialien, oder ein vergleichbares Implantat oder Transplantat, welches zum Ersatz einer insuffizienten Herzklappe geeignet ist. Somit bietet das System den Vorteil, in Abhängigkeit von den patientenspezifischen Bedingungen die optimale Ausführung einer Ersatzherzklappe aufweisen zu können. Des Weiteren weist die Ersatzherzklappe mindestens zwei Leaflets auf. Hinsichtlich des Ersatzes einer dreiteiligen Herzklappe ist auch der Einsatz mit mehr als zwei, insbesondere mit drei Leaflets, vorstellbar. Somit ist die Verwendung des erfindungsgemäßen Systems nicht nur auf den Ersatz einer insuffizienten, nativen Aortenklappe begrenzt, insbesondere nicht durch die Anzahl der Leaflets.

Die Leaflets der Ersatzherzklappe weisen in ihrer vorgesehenen Verwendung insbesondere zwei Positionen auf, die sie während der Systole und der Diastole des Herzens einnehmen. Mit dem Ziel eine native Herzklappe als biologisches Vorbild zu imitieren, ist eine äquivalente Übertragung der Funktionalität der Leaflets dem biologischen Vorbild entsprechend auch für den Ersatz der weiteren nativen Herzklappen vorstellbar. In einer ersten Position der Leaflets, während der Diastole des Herzens, wird die strömungsmäßige Verbindung zwischen linkem Ventrikel und Aorta vollständig abgeschlossen, so dass ein Blutrückfluss verhindert wird. Hierbei weisen die Kommissuren der Leaflets, die gefäßinnenseitig liegenden Kanten, Kontakt zueinander auf. Während der Systole des Herzens nehmen die Leaflets eine zweite, geöffnete Position ein, so dass das Blut vom Ventrikel aus in die Aorta gefördert werden kann. Die Kommissuren der Leaflets weisen in dieser zweiten Position keinen Kontakt zueinander auf.

Eine Ausführungsform des mindestens einen Stents weist vorzugsweise an der Innenseite und/oder Außenseite eine Beschichtung auf, die aus einer antimikrobiellen Substanz oder einem antimikrobiell wirkenden Trägermaterial besteht. Somit kann der mindestens eine Stent einen oder mehrere antimikrobielle Wirkstoffe freisetzen und eine verbesserte Integrierbarkeit des implantierten System im Kontakt mit der umliegenden Gefäßwand erzielen. Insbesondere bei Einsatz eines antimikrobiell wirkenden Trägermaterials ist eine Kombination mit weiteren Komponenten wie Antikoagulantien, weiteren antimikrobiellen Wirkstoffen wie Bakteriziden, etc. möglich, die an und/oder auf dem Trägermaterial platziert werden. Um derartige Beschichtungen erzeugen zu können, sind u.a. die Aufbringung einer Folie/Membran auf der Stent-Oberfläche sowie weitere physikalische und/oder chemische Ablagerungsverfahren zur Aufbringung einer Oberflächenbeschichtung für das erfindungsgemäße System anwendbar. Es ist somit möglich, an der Stent-Oberfläche eine komplexe Freisetzungskinetiken auch unterschiedlicher Wirkstoffe zu erzielen.

Eine Beschichtung der Stent-Oberfläche kann in einer weiteren Ausführungsform kontrolliert aktivierbar sein. Dabei tritt ein vorzugsweise antimikrobieller Effekt erst ein, wenn eine Aktivierung der Oberflächenbeschichtung erfolgt ist. Vorzugsweise kann eine derartige, kontrollierte Aktivierung der Oberflächenbeschichtung durch die Applikation von Ultraschall von außerhalb des Körpers des Patienten erfolgen, wobei mindestens eine, an spezielle Trägermedien gebundene, toxische Substanze, wie beispielsweise Kohlenstoffdioxid, an der Stent-Oberfläche freigesetzt wird.

Die Aufbringung kann auch in Form von Mikrofäden oder Vliesen erfolgen, aus denen der Wirkstoff freigesetzt werden kann, wobei es sich hierbei insbesondere um bioresorbierbare Polymermaterialien wie etwa Polylactide, Polyglycolide, Polylactone, Polydioxanone, Polyglycolen, Kombinationen und/oder chemischen Modifikationen daraus handeln kann, in die der oder die Wirkstoffe eingebracht wurden.

Alternativ oder zusätzlich hierzu weist das erfindungsgemäße System einen ersten Schürzenbereich innerhalb und einen zweiten Schürzenbereich außerhalb des vorgesehenen Stents auf, wobei mindestens eine auf- oder eingebrachte bzw. eingefüllte Substanz im ventrikelseitigen Retentionsbereich des Stents freigesetzt wird. Insbesondere wird unter dem ventrikelseitigen Retentionsbereich im Falle eines Aortenklappenersatzes der Retentionsbereich eines erfindungsgemäßen Stents bzw. Stent-Systems verstanden, der dem linken Ventrikel des Herzens zugewandt und dem aortaseitigen Retentionsbereich gegenübergestellt ist. Somit ergibt sich ein Blutfluss durch das erfindungsgemäße System, wobei mindestens eine Substanz am ventrikelseitigen Bluteingang des Systems freigesetzt wird, während der Blutstrom auf der Blutausgangsseite des erfindungsgemäßen Systems, am aortaseitigen Retentionsbereich, in die Aorta übergeht. Entsprechend der erfindungsgemäßen Verwendung des beanspruchten Systems, ist auch bei Ersatz anderer Herzklappen die Freisetzung der mindestens einen Substanz stets auf der Bluteingangsseite des erfindungsgemäßen Systems anzunehmen.

Die vorliegende Offenbarung zeigt ferner einen geeigneten Katheter zum Einführen eines Implantates in den Körper eines Patienten, insbesondere eines Implantates der zuvor genannten Art, welches zur Behandlung oder zum Ersatz einer an einer Entzündung, Thrombose oder Degeneration erkrankten Herzklappe dient. Der Katheter weist hierzu eine Katheterspitze auf, welche über eine Handhabe des Katheters derart manipulierbar ist, dass das Implantat sequenziell, das heißt schrittweise, von der Katheterspitze freigebbar ist.

Gemäß einem weiteren Aspekt der vorliegenden Erfindung betrifft diese ein entsprechendes System zur Behandlung oder zum Ersatz einer an einer Entzündung und/oder Infektion erkrankten Herzklappe, wobei dieses System ein Implantat gemäß der vorliegenden Erfindung sowie einen Katheter der zuvor genannten Art aufweist. Hierbei ist das Implantat vorzugsweise ausgebildet, in einem in Längsrichtung des Implantates gestreckten und im Hinblick auf die Radialrichtung des Implantates komprimierten oder reduzierten Zustand in der Katheterspitze des Einführ-Katheters aufnehmbar zu sein. Andererseits ist die Katheterspitze des Einführ-Katheters vorzugsweise ausgebildet, das Implantat in seinem in Längsrichtung des Implantates gestreckten und im Hinblick auf die Radialrichtung des Implantates reduzierten Zustand aufzunehmen.

Indem gemäß diesen Ausführungsformen das Implantat in einem gestreckten Zustand in der Katheterspitze aufnehmbar ist, kann der effektive Außendurchmesser der Katheterspitze und insbesondere auch eines Kathetersystems, welches die Katheterspitze mit der Handhabe des Katheters verbindet, einen besonders geringen Durchmesser aufweisen. Gemäß bevorzugten Realisierungen liegt der Durchmesser des Katheters bei 18F oder darunter.

Bei dem nicht beanspruchten Verfahren zur Behandlung einer an einer Entzündung und/oder Infektion erkrankten Herzklappe ist insbesondere vorgesehen, dass zunächst hyperplastische Gewebeveränderungen insbesondere in Gestalt einer Herzklappenvegetation bei der erkrankten (nativen) Herzklappe kompartimentiert werden, und zwar insbesondere indem die hyperplastischen Gewebeveränderungen zumindest bereichsweise von einer geeigneten Fangeinrichtung, die in dem Körper des Patienten zu implantieren ist, aufgenommen und/oder umgriffen werden. Anschließend kann eine Ersatzherzklappe implantiert werden, um die Funktion der erkrankten, nativen Herzklappe zu ersetzen. Ferner kann das Verfahren den weiteren Verfahrensschritt der Freigabe von antimikrobiellen Wirkstoffen aufweisen, wobei die Freigabe vorzugsweise in situ erfolgt.

Das erfindungsgemäße Implantat ist insbesondere ausgebildet, im Implantationsort an der erkrankten nativen Herzklappe zeitlich gesehen nacheinander schrittweise zu expandieren, wobei in einem ersten Schritt die Fangeinrichtung und in einem zweiten Schritt die Ersatzherzklappe expandiert.

Alternativ hierzu ist das Implantat ausgebildet, im Implantationsort an der erkrankten nativen Herzklappe zeitlich gesehen nacheinander schrittweise zu expandieren, wobei in einem ersten Schritt die Ersatzherzklappe und in einem zweiten Schritt die Fangeinrichtung expandiert.

Gemäß bevorzugten Realisierungen weist das Implantat eine insbesondere separat von der Fangeinrichtung und Herzklappe implantierbare Struktur auf, welche insbesondere einen im Wesentlichen ringförmigen selbstexpandierenden Aufbau aufweist und in die Taschen der nativen Herzklappe einsetzbar ist und als Widerlager insbesondere für die Fangeinrichtung oder den Aufspannbereich des Implantats dient.

Die Erfindung betrifft ferner einen Katheter zum Einführen eines Implantates in den Körper eines Patienten, insbesondere eines Implantates zur Behandlung oder zum Ersatz einer an einer entzündeten, thrombosierten oder degenerierten Herzklappe der zuvor genannten Art, wobei der Katheter eine Katheterspitze aufweist, welche über eine Handhabe des Katheters derart manipulierbar ist, dass das Implantat schrittweise von der Katheterspitze freisetzbar ist.

Darüber hinaus betrifft die Erfindung ein System zur Behandlung oder zum Ersatz einer an einer entzündeten, thrombosierten oder degenerierten Herzklappe mit einem Implantat der erfindungsgemäßen Art und einem Katheter der zuvor genannten Art, wobei das Implantat ausgebildet ist, insbesondere in einem in Längsrichtung des Implantates gestreckten und im Hinblick auf die Radialrichtung des Implantates reduzierten Zustand in der Katheterspitze aufnehmbar zu sein, und wobei die Katheterspitze ausgebildet ist, das Implantat insbesondere in seinem in Längsrichtung des Implantates gestreckten und im Hinblick auf die Radialrichtung des Implantates reduzierten Zustand aufzunehmen.

Gemäß Ausführungsformen weist das System ein zusätzliches und/oder unabhängiges insbesondere zumindest im Wesentlichen ringförmiges, selbstexpandierendes Implantat auf, welches hierin auch als "Widerlager-Implantat" bezeichnet wird und ausgebildet ist, insbesondere als Widerlager für das Implantat zu dienen, und welches insbesondere über eine Streckung in einer radialen Richtung faltbar und in gefalteter Form in einer Katheterspitze aufnehmbar und derart am Implantationsort implantierbar ist, dass im implantierten Zustand des Implantates zwischen dem Implantat und dem zusätzlichen und/oder unabhängigen Implantat zumindest bereichsweise mindestens ein Klappensegel der zu behandelnden Herzklappe aufnehmbar ist. Das Widerlager-Implantat kann ausgebildet sein, im komprimierten Zustand in der Katheterspitze so aufnehmbar zu sein, dass das Widerlager-Implantat im komprimierten Zustand nicht koaxial, sondern orthogonal zur Katheterspitzenachse ausgerichtet ist und mittels einer oder mehrerer druckstabiler Verbindungen auch schrittweise am Implantationsort freisetzbar ist.

Ferner betrifft die Beschreibung ein Verfahren zur Behandlung oder zum Ersatz einer an einer entzündeten, thrombosierten oder degenerierten Herzklappe, wobei das Verfahren den Verfahrensschritt der Kompartimentierung von Gewebeveränderungen oder Gewebeauflagerungen insbesondere in Gestalt von Herzklappenvegetationen oder -auflagerungen aufweist, und zwar insbesondere indem die Gewebeveränderungen oder Gewebeauflagerungen zumindest bereichsweise von einer Fangeinrichtung aufgenommen und/oder umgriffen werden.

Das Verfahren weist vorzugsweise ferner den Verfahrensschritt des Implantierens einer Ersatzherzklappe auf zum Ersatz der erkrankten nativen Herzklappe, wobei der Schritt der Kompartimentierung und der Schritt der Implantierung der Ersatzherzklappe und des Widerlagers vorzugsweise zeitlich gesehen nacheinander ausgeführt werden.

Insbesondere weist das Verfahren ferner den Verfahrensschritt der Freigabe von antimikrobiellen, antithrombotischen und zellwachstumshemmenden Wirkstoffen auf, wobei die Freigabe in-situ erfolgt.

Nachfolgend werden exemplarische Ausführungsformen der Erfindung anhand der beiliegenden Zeichnungen näher beschrieben.

Es zeigen:
- FIG. 1A und 1B: schematisch und in einer Schnittansicht eine an einer Endokarditis erkrankte Aortenklappe mit bakterieller Vegetation, wobei FIG. 1A die Aortenklappe im geschlossenen Zustand und FIG. 1B die Aortenklappe im geöffneten Zustand zeigt;

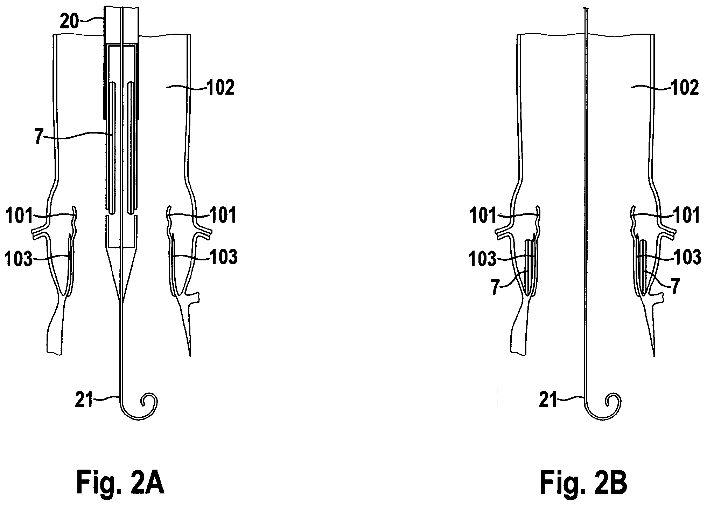

- FIG 2A und 2B: schematisch und in einer Schnittansicht das Einbringen (FIG. 2A) und die finale Position (FIG. 2B) eines als Widerlager für einen weiteren Stent dienendes, mit Wirkstoff beschicktes Implantat;

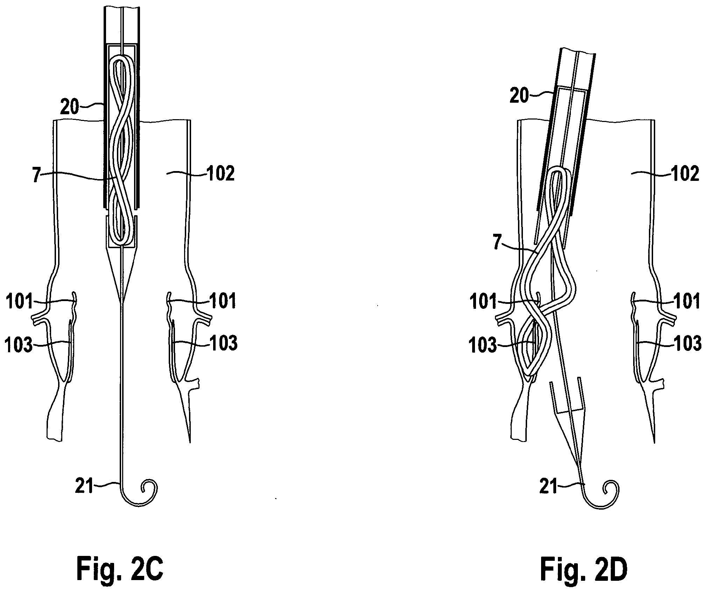

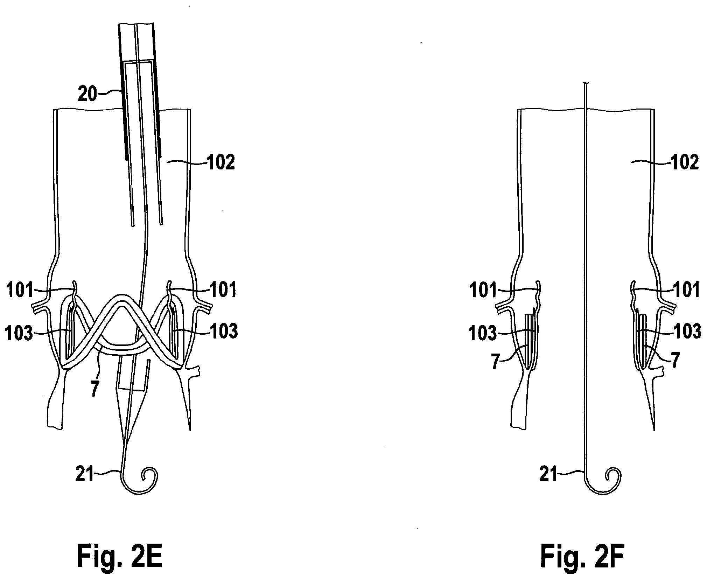

- FIG 2C bis 2F: schematisch und in einer teilgeschnittenen Ansicht (FIG. 2C bis 2E) bzw. geschnittenen Ansicht (FIG. 2F) das Einführen orthogonal zur Katheterachse, das Herausschieben aus dem Katheter und das Platzieren in hinter den Segeln der Aortenklappe eines als Widerlager für einen weiteren Stent dienendes, mit Wirkstoff beschicktes Implantats;
- FIG. 3A bis 3F: schematisch und in einer Schnittansicht das Einbringen, Platzieren und Freisetzen einer ersten exemplarischen Ausführungsform zur Behandlung und zum Ersatz einer an einer Endokarditis erkrankten Aortenklappe;
- FIG. 4A bis 4D: schematisch und in einer Schnittansicht das Einbringen, Platzieren und Freisetzen einer zweiten exemplarischen Ausführungsform zur Behandlung und zum Ersatz einer an einer Endokarditis erkrankten Aortenklappe; und
- FIG. 5A bis 5E: schematisch und in einer Schnittansicht das Einbringen, Platzieren und Freisetzen einer dritten exemplarischen Ausführungsform zur Behandlung und zum Ersatz einer an einer Endokarditis erkrankten Aortenklappe.

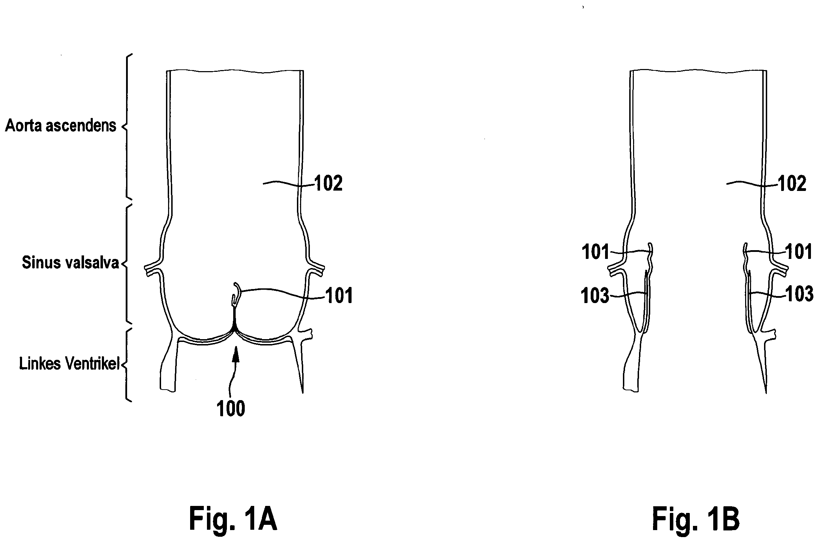

In FIG. 1 ist schematisch und in einer Schnittansicht die Anatomie einer an einer Endokarditis erkrankten Aortenklappe 100 gezeigt, wobei in FIG 1A die Aortenklappe geschlossen und in FIG 1B die Aortenklappe geschlossen dargestellt ist.

Kurz zusammengefasst ist die Aortenklappe 100 eine der vier Herzklappen. Sie liegt in der Aorta 102, direkt an deren Ursprung aus der linken Herzkammer und verhindert den Rückfluss des Blutes zu Beginn der Erschlaffungsphase (Diastole) des Herzens.

Die Aortenklappe 100 besteht als Taschenklappe zumeist aus drei halbmondförmigen Taschen. Die Klappe liegt mit ihren Ausbuchtungen (Sinus) im Anfangsteil der aufsteigenden Aorta 102 (Aorta ascendens). Auf Deutsch werden die Taschen entsprechend der Abgänge der beiden Herzkranzarterien aus den zugehörigen Sinus bezeichnet: rechtskoronare Tasche am Abgang der rechten Herzkranzarterie, linkskoronare Tasche am Abgang der linken Herzkranzarterie und akoronare Tasche (Sinus ohne abgehende Herzkranzarterie). Bei einer Aortenklappeninsuffizienz und/oder einer Aortenklappen-Endokarditis ist es häufig erforderlich, die native Aortenklappe 100 durch eine Ersatzherzklappe 10 auszutauschen.

Eine Aortenklappen-Endokarditis kann durch zahlreiche Mikroorganismen verursacht werden. Besonders grammpositive Bakterienspezies wie z.B. Streptokokken, Enterokokken und Staphylokokken treten häufig als humanpathogene Keime bei infektiöser Endokarditis auf. Wenn diese im Zuge einer Bakteriämie das Endokard beispielsweise der nativen Aortenklappe 100 besiedeln, entsteht eine infektiöse Endokarditis, bei welcher es sich um eine Entzündung der Herzinnenhaut (Endokard) handelt. Unbehandelt ist der Krankheitsverlauf meist tödlich.

Im Rahmen einer bakteriellen Endokarditis werden ferner Gewebeauflagerungen 101 der Herzklappen 100 durch Bakterien, deren Stoffwechselprodukte sowie weiteren Bestandteilen des menschlichen Organismus gebildet. Derartige Gewebeauflagerungen 101 werden auch als "Herzklappenvegetation" bezeichnet. Als Voraussetzung für die bakterielle Besiedelung können sogenannte nicht bakterielle thrombotische Vegetationen angesehen werden. Dabei handelt es sich um Thrombozyten, die sich auf geschädigtem Endothel der Herzklappen 100 festsetzen.

Bei einer Herzklappen-Endokarditis ist die Herzklappenvegetation 101 insbesondere in Gestalt von faden- oder membranartigen Strukturen an den nativen Herzklappen 100 ausgebildet, welche bis über 25 mm lang sein können.

Hierbei besteht die Gefahr, dass die Vegetationen 101 durch das pumpende Herz losgerissen werden und bei ihrem Fluss durch den Blutkreislauf Blutgefäße in den Organen verstopfen. Daraus entstehende, gefürchtete Komplikationen können u.a. sein: ein Schlaganfall oder eine Nierenembolie, wobei vor allem der Schlaganfall gefürchtet ist, da bei ihm ein großes Risiko von Entzündungen des Gehirns oder der Hirnhäute besteht.

Eine weitere Komplikation ist darin zu sehen, dass die Gefahr der Verschleppung von Keimen in andere Organe besteht, wo sich dann Abszesse bilden können. Im Zuge einer Blutvergiftung und dem septischen bzw. toxischen Schock bei giftbildenden Bakterien kann es zu einem akuten Organausfall kommen (beispielsweise Nieren-, Leber- und/oder Lungenversagen).

Um das von der Herzklappenvegetation 101 ausgehende Risiko bei der Behandlung einer Aortenklappen-Endokarditis zu minimieren, ist erfindungsgemäß vorgesehen, dass bei einem im Rahmen des Ersatzes einer an einer Endokarditis erkrankten Herzklappe 100 die Herzklappenvegetation 101 entsprechend kompartimentiert wird. Dies kann vorzugsweise vor dem Ersatz der nativen Herzklappe 100 durch eine Herzklappenprothese 10, oder aber auch während des Ersatzes oder im Anschluss des Ersatzes der erkrankten nativen Herzklappe 100 erfolgen.

Zur Kompartimentierung der Herzklappenvegetation 101 kommt ein Implantat 1 zum Einsatz, welches eine Fangeinrichtung 2 aufweist, wobei diese Fangeinrichtung 2 in einem komprimierten Zustand minimal-invasiv in den Körper des Patienten einführbar und im Implantationsort an der erkrankten Herzklappe 100 expandierbar ist. Die Fangeinrichtung 2 ist ausgebildet, zumindest im implantierten und expandierten Zustand Gewebeauflagerungen 101 (Herzklappenvegetation) zumindest bereichsweise zu umgreifen und/oder aufzunehmen und somit zu kompartimentieren.

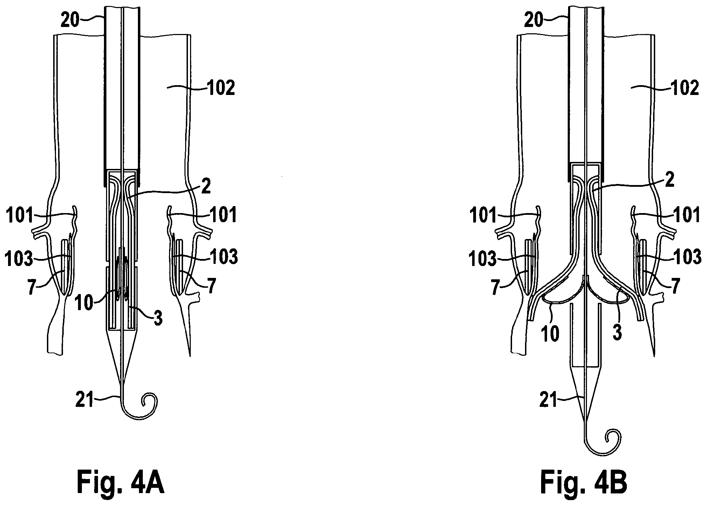

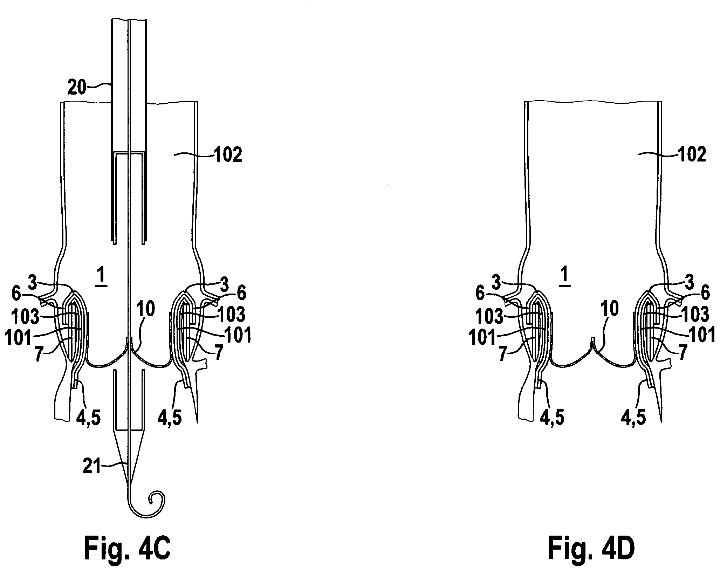

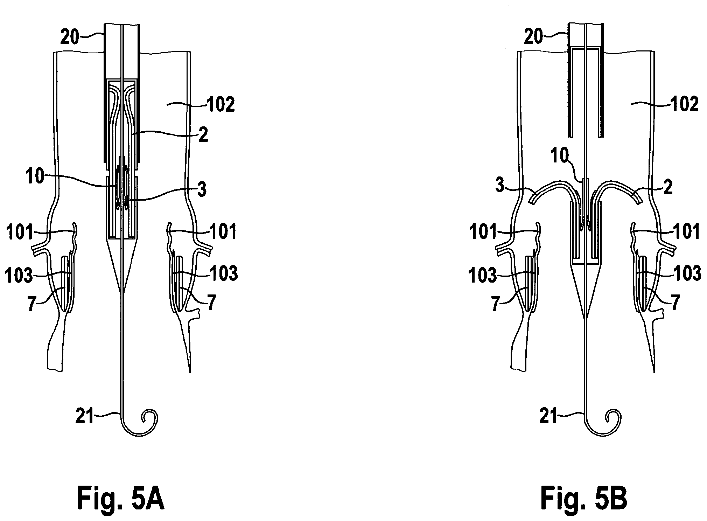

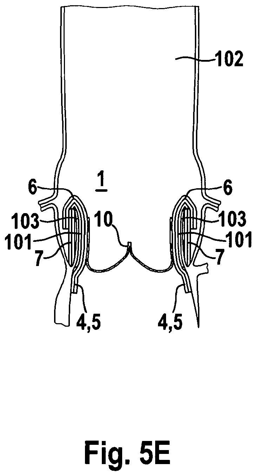

Bei dem in FIG. 3A bis 3F, FIG 4A bis 4D und in FIG. 5A bis 5E gezeigten Ausführungsformen weist das Implantat 1 mit der Fangeinrichtung 2 ferner eine entsprechende Ersatzherzklappe 10 auf, bei welcher es sich um eine entweder selbst-expandierbare oder ballon-expandierbare Ersatzklappe handelt, die in einem komprimierten Zustand insbesondere minimal-invasiv in den Körper des Patienten einführbar und im Implantationsort an der erkrankten Herzklappe 100 derart expandierbar ist, dass die Ersatzherzklappe 10 zumindest im Wesentlichen die Funktion einer nativen Herzklappe 100 übernimmt.

Der Ersatzherzklappe 10 ist vorzugsweise ein Stent 3 zugeordnet, um die Ersatzherzklappe 10 abzustützen und zu tragen. Der der Ersatzklappe 10 zugeordnete Stent 3 ist ausgelegt, die erkrankte native Herzklappe 100 bzw. die Herzklappensegel 103 der erkrankten, nativen Herzklappe 100 radial zu verdrängen, um die Ersatzherzklappe 10 an dessen Stelle aufzuspannen und eine fehlerfreie Klappenfunktion während der Systole und Diastole des Herzens zu ermöglichen.

Der der Ersatzklappe 10 zugeordnete Stent 3 ist insbesondere strukturell ausgebildet, damit er währen des periodischen Herzschlages der Ersatzherzklappe 10 einen sicheren Halt bieten kann, so dass das Implantat 1 nicht aufgrund wechselnder Druckverhältnisse im Herzen von dem nativen biologischen Gewebe, insbesondere von der Gefäßwand, abgelöst und vom Implantationsort ausgeschwemmt werden kann.

Der mindestens eine der Ersatzklappe 10 zugeordnete Stent 3 dient vorzugsweise ferner als Verankerungsstruktur für die Fangeinrichtung 2 des Implantats 1. Selbstverständlich ist es in diesem Zusammenhang aber auch denkbar, für diese Funktionen (Träger- und Stützstruktur der Ersatzherzklappe 10 und Verankerungsstruktur der Fangeinrichtung 2) mehrere Stents 3 oder Stent-Systeme zu verwenden, die geeignet miteinander verbunden oder verbindbar sind.

Der oder die Stents 3 des erfindungsgemäßen Implantats 1 können per Ballon-Expansion mit Hilfe eines Ballonkatheters expandiert und am Implantationsort positioniert werden. Dabei wird der komprimierte und im Katheter verborgene Stent 3 durch einen mit Flüssigkeit oder Gas zu füllenden Katheterballon expandiert.

Alternativ kann der mindestens eine Stent 3 des erfindungsgemäßen Implantats 1 ein selbst-expandierbarer Stent 3 sein. Insbesondere besteht der Stent 3 hierzu aus einer Formgedächtnislegierung, vorzugsweise aus Nitinol. Neben dem Formgedächtniseffekt bei einer spezifischen Sprungtemperatur nahe der Körpertemperatur weist Nitinol gleichfalls Superelastizität, Biokompatibilität und Korrosionsbeständigkeit auf. So wird Nitinol bereits vielfach in der Medizintechnik angewendet.

Insbesondere die Superelastizität ist vorteilhaft hinsichtlich der komprimierten Zuführungsform eines Stents im Transkatheter-Verfahren und der Expansion am Implantationsort.

Neben den zwei separat ausgeführten Expansionsverfahren ist gleichfalls auch eine Kombination beider Verfahren möglich. Insbesondere die radiale Vorspannkraft des mindestens einen Stents 3 des Implantats 1 kann nach der Selbstexpansion durch eine Ballonexpansion noch zusätzlich gesteigert werden, wobei wiederum eine höhere Stabilität des erfindungsgemäßen Implantats 1 im implantierten Zustand erreicht werden.

Die an dem mindestens einen Stent 3 befestigte Ersatzherzklappe 10 kann eine Perikardklappe sein, eine biologische (beispielsweise Schweine- oder Rinder-) Herzklappe, eine künstliche Herzklappe 100, vorzugsweise aus bio-kompatiblen Materialien, oder ein vergleichbares Implantat oder Transplantat, welches zum Ersatz einer an Endokarditis erkrankten Herzklappe 100 geeignet ist. Somit bietet das erfindungsgemäße Implantat 1 den Vorteil, in Abhängigkeit von den Patienten spezifischen Bedingungen die optimale Ausführung einer Ersatzherzklappe 10 aufweisen zu können.

Die Ersatzherzklappe 10 weist mindestens zwei Herzklappensegel 103 (Englisch: Leaflets) auf. Hinsichtlich des Ersatzes einer dreiteiligen Herzklappe 100 ist auch der Einsatz mit mehr als zwei, insbesondere mit drei Leaflets 103, vorstellbar. Somit ist die Verwendung des erfindungsgemäßen Implantats 1 nicht nur auf die Behandlung und den Ersatz einer an einer Endokarditis erkrankten nativen Aortenklappe 100 begrenzt, insbesondere nicht durch die Anzahl der Leaflets 103.

Die Leaflets 103 der Ersatzherzklappe 10 weisen in ihrer vorgesehenen Verwendung insbesondere zwei Positionen auf, die sie währen der Systole und der Diastole des Herzens einnehmen. Mit dem Ziel, eine native Herzklappe 100 als biologisches Vorbild zu imitieren, ist eine äquivalente Übertragung der Funktionalität der Leaflets 103 dem biologischen Vorbild entsprechend auch für den Ersatz der weiteren nativen Herzklappen 100 vorstellbar. In einer ersten Position der Leaflets 103, während der Diastole des Herzens, wird die strömungsmäßige Verbindung zwischen dem linken Ventrikel und der Aorta 102 vollständig abgeschlossen, so dass ein Blutrückfluss verhindert wird. Dabei weisen die Kommissuren der Leaflets 103, d.h. die gefäßinnenseitig liegenden Kanten, Kontakt zueinander auf.

Während der Systole des Herzens nehmen die Leaflets 103 eine zweite, geöffnete Position ein, so dass das Blut vom Ventrikel aus in die Aorta 102 gefördert werden kann. Die Kommissuren der Leaflets 103 weisen in dieser zweiten Position keinen Kontakt zueinander auf.

Die Fangeinrichtung 2 des erfindungsgemäßen Implantats 1 kann eine Verankerungsstruktur in Gestalt eines Stents 3 aufweisen, wobei dieser Stent 3 gleichwohl als Träger und Stützstruktur der Ersatzherzklappe 10 dienen kann. Auf diese Weise kann auf die Fangeinrichtung 2 im Implantationsort geeignet fixiert und positioniert werden.

Mit der Verankerungsstruktur 3 ist eine Fangstruktur 4 verbunden, welche in Blutstromrichtung gesehen sich von der Verankerungsstruktur 3 stromabwärts in ein strömungsmäßig mit der erkrankten Herzklappe 100 verbundenes Gefäß 102, insbesondere in die Aorta, erstreckt und im expandierten Zustand mindestens einen Aufspannbereich 5 aufweist, welcher ausgebildet ist, im expandierten und implantierten Zustand der Fangeinrichtung 2 mit mindestens einem Herzklappensegel 103 der erkrankten Herzklappe 100 derart wechselzuwirken, dass der mindestens eine Herzklappensegel 103 zwischen Verankerungsstruktur 3 und Fangstruktur 4 positioniert wird.

Der Aufspannbereich 5 der Fangeinrichtung 2 kann mindestens einen Aufspannarm oder Aufspannbügel aufweisen, welcher ausgebildet ist, bei der Expansion der Fangeinrichtung 2 im Implantationsort zumindest bereichsweise in radialer Richtung zu expandieren.

Gemäß Ausführungsformen kann der Aufspannbereich 5 an einem der Verankerungsstruktur 3 abgewandten, distalen Endbereich mindestens einen zumindest im Wesentlichen in Richtung der Verankerungsstruktur 3 zeigenden Bereich 6 aufweisen, um eine Umgreifung oder eine Aufnahme der Herzklappenvegetation 101 zu optimieren und kraft- oder formschlüssig mit einer erfindungsgemäßen Struktur 7 (z.B. Widerlager-Implantat) wechselzuwirken.

Bei der Behandlung bzw. dem Ersatz einer an einer Endokarditis erkrankten Herzklappe 100, insbesondere Aortenklappe 100, kann vor dem Einführen des Implantats 1 mit der Fangeinrichtung 2 und der Ersatzherzklappe 10 zunächst eine Struktur 7 eingesetzt werden, welche einen im Wesentlichen ringförmigen Aufbau aufweist und sich beispielsweise in den Taschen der nativen Herzklappe 100 abstützt und diese ausfüllen kann. Diese Struktur 7 dient als Widerlager für das im Anschluss daran zu implantierende Implantat 1. Vorzugsweise weist die ringförmige Widerlagerstruktur 7 eine Form und Höhe in Längsrichtung auf, welche im implantierten Zustand nicht die Öffnungen der Koronararterien blockiert.

Das erfindungsgemäße Implantat 1 kann - wie in den Zeichnungen dargestellt - als ein zusammenhängendes System im Rahmen einer Einführprozedur schrittweise implantiert werden. Allerdings ist es aber auch denkbar, das Implantat 1 und die Struktur 7 über eine Reihe von separaten Einführprozeduren nacheinander zu implantieren und insbesondere dann, d.h. im implantierten Zustand die Komponenten des Implantats 1, insbesondere die Fangeinrichtung 2 und die Ersatzherzklappe 10 bzw. ein der Ersatzherzklappe 10 zugeordneter Stent 3, zu verbinden.

Bei der in FIG. 3A bis 3F gezeigten Ausführungsform wird das Implantat zeitlich gesehen schrittweise implantiert, wobei in einem ersten Schritt die Fangeinrichtung expandiert und nach unten geschoben und mit dem Widerlager-Implantat 7 kraft- oder formschlüssig verbunden wird und in einem zweiten Schritt die Herzklappe expandiert und freigesetzt wird.

Bei der in FIG. 4A bis FIG. 4D gezeigten Ausführungsform wird das Implantat 1 zeitlich gesehen nacheinander schrittweise expandiert, wobei in einem ersten Schritt die Ersatzherzklappe 10 implantiert und expandiert wird, und in einem nachfolgenden zweiten Schritt die Fangeinrichtung 2 expandiert.

Hingegen ist bei der in FIG. 5A bis FIG. 5E gezeigten Einführprozedur vorgesehen, dass zunächst die Fangeinrichtung 2 expandiert und anschließend die Ersatzherzklappe 10.

Bei beiden Einführprozeduren wird das in einer Katheterspitze eines Einführkathetersystems 20 im komprimierten Zustand aufgenommene Implantat 1 über einen transfemoralen Zugang zum Implantationsort vorgebracht. Grundsätzlich ist es aber auch denkbar, wenn das Implantat 1 über einen transapikalen Weg, d.h. von der Herzspitze kommend, eingeführt wird.

Zur Implantation wird zunächst vorzugsweise das ringförmige Widerlager 7 in die Taschen der nativen Herzklappe 100 eingeführt. Allerdings ist die Erfindung nicht auf das Vorsehen eines solchen Widerlagers 7 beschränkt.

Eine Ausführungsform des Widerlager-Implantates 7 sieht vor, dieses koaxial im Katheter, d.h. mit radialer Kompression und dadurch axialer Streckung über einen Katheter einzuführen und dann radial (selbst) zu expandieren. Eine andere Ausführungsform des Widerlager-Implantates 7 sieht vor, dieses nicht koaxial, sondern zumindest im Wesentlichen gefaltet und orthogonal zur Katheterachse einzuführen, um durch bei gleichem Katheterdurchmesser ein größeres Volumen einzubringen.

Anschließend erfolgt die Implantation des erfindungsgemäßen Implantats 1 über einen Katheter 20, in dessen Katheterspitze das Implantat 1 in einem komprimierten Zustand aufgenommen ist. Vorzugsweise ist das Implantat 1 in einem in Längsrichtung des Implantats 1 gestreckten und im Hinblick auf die Radialrichtung des Implantats 1 reduzierten Zustand in der Katheterspitze des Einführkathetersystems 20 aufgenommen, um so den Durchmesser des Kathetersystems 20 minimieren zu können.

Darüber hinaus kommt zur Implantation vorzugsweise ein Führungsdraht 21 zum Einsatz, um die Einfuhr des Katheters zu vereinfachen und sicherer zu gestalten.

Bei der in FIG. 4A bis 4C gezeigten Einführprozedur wird die Katheterspitze soweit in Richtung des Ventrikels verschoben, dass die in der Katheterspitze aufgenommene Ersatzherzklappe 10 in Höhe der nativen Herzklappe 100 vorliegt. Anschließend findet die Expansion eines der Ersatzherzklappe 10 zugeordneten Stents 3, an welchem die Ersatzherzklappe 10 geeignet befestigt ist, statt.

Dies erfolgt, in dem die Katheterspitze geeignet manipuliert wird, um einen hülsenförmigen Bereich, welcher zumindest bereichsweise die Katheterspitze ausbildet und das Implantat 1 im komprimierten Zustand hält, in proximaler Richtung verschoben wird. Aufgrund dieser Verschiebung wird ein Teil des Implantats 1 freigegeben, und insbesondere der der Ersatzherzklappe 10 zugeordnete Stent 3 mit der daran befestigten Ersatzherzklappe 10.

Bei der Expansion des der Ersatzherzklappe 10 zugeordneten Stents 3 drückt dieser die nativen Herzklappensegel 103 der erkrankten Herzklappe 100 in radialer Richtung, wobei die optional zuvor in die Taschen der nativen Herzklappe 100 eingesetzte ringförmige Struktur 7 als Widerlager dient.

In dem in FIG. 4B gezeigten Zustand ist es insbesondere möglich, die korrekte Positionierung der Ersatzherzklappe 10 sowie deren einwandfreie Funktionalität zu überprüfen. Sollte sich hierbei zeigen, dass die Ersatzherzklappe 10 nicht an richtiger Stelle positioniert ist, so kann dies ohne Weiteres problemlos korrigiert werden. Sollte die Ersatzherzklappe 10 hingegen nicht ordnungsgemäß funktionieren, so kann diese wieder in die Katheterhülse zurückgeführt und das Implantat 1 wieder aus dem Körper des Patienten entfernt werden.

Sobald die richtige Positionierung und die Funktionsweise der Ersatzherzklappe 10 verifiziert wurden, kann dann die Fangeinrichtung 2 freigegeben werden, und zwar indem ein entsprechend zweiter hülsenförmiger Bereich der Katheterspitze distal verschoben wird. Die Fangeinrichtung 2 ist vorzugsweise derart "programmiert", dass sie bei ihrer Expansion die Herzklappenvegetation 101 umschließt und so aus dem Hauptblutstrom nimmt.

Bei den in FIG. 3A bis FIG. 3F und FIG. 5A bis FIG. 5E gezeigten alternativen Einführprozeduren wird zunächst die Fangeinrichtung 2 von der Katheterspitze freigegeben. Anschließend erfolgt die Freigabe der Ersatzherzklappe 10 bzw. des der Ersatzherzklappe 10 zugeordneten Stents 3.

Diese Einführprozedur weist den Vorteil auf, dass die Herzklappenvegetation 101 bereits kompartimentiert ist, bevor die Ersatzherzklappe 10 expandiert; allerdings erfordert die in FIG. 5A bis 5E gezeigte Einführprozedur, dass die Katheterspitze weiter in Richtung Ventrikel eingeführt werden muss.

Bei den in FIG. 3A bis FIG 3F und in FIG. 5A bis 5E gezeigten Einführprozeduren expandiert bei der Freigabe der Fangeinrichtung 2 zunächst ein Aufspannbereich 5, wobei dieser Aufspannbereich 5 mit einer Verankerungsstruktur 3 des Implantats 1 (noch nicht implantiert) verbunden ist.

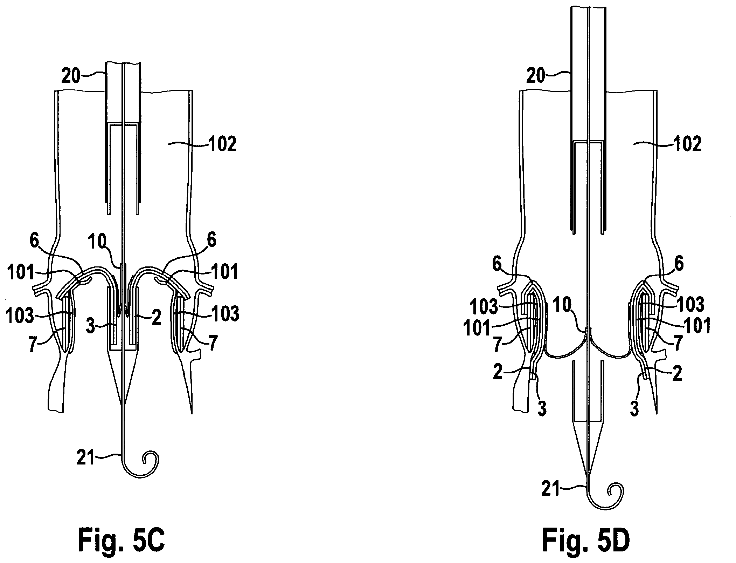

Den Darstellungen in FIG. 5B und 5C ist zu entnehmen, dass der Aufspannbereich 5 an einem der Verankerungsstruktur 3 abgewandten distalen Endbereich mindestens einen im Wesentlichen in Richtung der Verankerungsstruktur 3 zeigenden Bereich 6 aufweist, welcher das Einfangen und Umschließen der Herzklappenvegetation 101 wesentlich vereinfacht.

Nach der vollständigen Freigabe und Expansion der Fangeinrichtung 2 (vgl. FIG. 5D sowie auch FIG. 3C) wird durch Manipulation eines vorderen hülsenförmigen Bereichs der Katheterspitze in proximaler Richtung die Ersatzherzklappe 10 bzw. ein der Ersatzherzklappe 10 zugeordneter Stent 3 freigegeben.

Das Implantat 1 und/oder das ringförmige und in die Taschen der nativen Herzklappe 100 eingesetzte Widerlager 7 sind/ist insbesondere ausgebildet, in situ einen oder mehrere Wirkstoffe, insbesondere antibakterielle, antithrombotische und zellwachstumshemmende Verbindungen, freizusetzen.

### Bezugszeichenliste

- 1: Implantat
- 2: Fangeinrichtung
- 3: Stent
- 4: Fangstruktur
- 5: Aufspannbereich
- 6: Zur Widerlager-Struktur zeigender Bereich der Fangstruktur
- 7: Widerlager-Implantat/Widerlager-Struktur

- 10: Herzklappenprothese

- 20: Katheter
- 21: Führungsdraht

- 100: Herzklappe
- 101: Herzklappenvegetation/Gewebeauflagerung
- 102: Aorta
- 103: (natives) Herzklappensegel

## Patentansprüche

1. Implantat (1) zur Behandlung und/oder zum Ersatz einer an einer Entzündung und/oder Infektion erkrankten Herzklappe (100), wobei das Implantat (1) eine Fangeinrichtung (2) aufweist, welche in einem komprimierten Zustand minimal-invasiv in den Körper des Patienten einführbar und im Implantationsort an der erkrankten Herzklappe (100) expandierbar ist, wobei die Fangeinrichtung (2) ausgebildet ist, zumindest im implantierten und expandierten Zustand Gewebeveränderungen oder Gewebeauflagerungen (101) in Gestalt von Herzklappenvegetationen oder - auflagerungen zu kompartimentieren, indem die Gewebeveränderung oder -auflagerung (101) zumindest bereichsweise von der Fangeinrichtung (2) umgriffen und/oder aufgenommen wird,
wobei die Fangeinrichtung (2) eine Verankerungsstruktur (3) aufweist zum Fixieren und/oder Positionieren der Fangeinrichtung (2) am Implantationsort, und wobei die Fangeinrichtung (2) eine mit der Verankerungsstruktur (3) verbundene Fangstruktur (4) aufweist, welche sich in Blutstromrichtung gesehen von der Verankerungsstruktur (3) stromabwärts in ein mit der erkrankten Herzklappe (100) strömungsmäßig verbundenes Gefäß (102) erstreckt und im expandierten Zustand mindestens einen Aufspannbereich (5) aufweist, welcher ausgebildet ist, im expandierten und implantierten Zustand der Fangeinrichtung (2) mit mindestens einem Herzklappensegel (103) der erkrankten Herzklappe (100) derart wechselzuwirken, dass mindestens ein Herzklappensegel (103) in Richtung einer Gefäßwand gedrückt und mindestens teilweise von der Fangeinrichtung (2) umschlossen wird.

2. Implantat (1) nach Anspruch 1,
wobei der mindestens eine Aufspannbereich (5) mindestens einen Aufspannarm oder Aufspannbügel aufweist, welcher ausgebildet ist, bei der Expansion der Fangeinrichtung (2) im Implantationsort zumindest bereichsweise in radialer Richtung zu expandieren, wobei der Aufspannbereich (5) vorzugsweise ausgebildet ist, an einem der Verankerungsstruktur (3) abgewandten distalen Endbereich mit einer weiteren zu implantierenden Widerlagerstruktur (7) zu interagieren.

3. Implantat (1) nach Anspruch 1 oder 2,
wobei das Implantat (1) ferner eine expandierbare Ersatzherzklappe (10) aufweist, welche in einem komprimierten Zustand insbesondere minimal-invasiv in den Körper des Patienten einführbar und im Implantationsort an der erkrankten Herzklappe (100) derart expandierbar ist, dass die Ersatzherzklappe (10) die Funktion einer nativen Herzklappe (100) übernimmt, wobei die Ersatzherzklappe (10) und die Fangeinrichtung (2) vorzugsweise miteinander verbunden oder verbindbar sind, vorzugsweise über eine gemeinsame Verankerungsstruktur (3), welche ausgebildet ist, insbesondere im Bereich der Wurzeln der Herzklappensegel (103) der nativen Herzklappe (100) positioniert und fixiert zu werden.

4. Implantat (1) nach Anspruch 3,
wobei das Implantat (1) ausgebildet ist, im Implantationsort an der erkrankten nativen Herzklappe (100) zeitlich gesehen nacheinander schrittweise zu expandieren, wobei in einem ersten Schritt die Fangeinrichtung (2) und in einem zweiten Schritt die Ersatzherzklappe (10) expandiert; oder
wobei das Implantat (1) ausgebildet ist, im Implantationsort an der erkrankten nativen Herzklappe (100) zeitlich gesehen nacheinander schrittweise zu expandieren, wobei in einem ersten Schritt die Ersatzherzklappe (10) und in einem zweiten Schritt die Fangeinrichtung (2) expandiert.

5. Implantat (1) nach einem der Ansprüche 1 bis 4,
wobei das Implantat (1) ferner eine insbesondere separat von der Fangeinrichtung (2) und Herzklappe (100) implantierbare Struktur (7) aufweist, welche insbesondere einen im Wesentlichen ringförmigen selbstexpandierenden Aufbau aufweist und in die Taschen der nativen Herzklappe (100) einsetzbar ist und als Widerlager insbesondere für die Fangeinrichtung (2) oder den Aufspannbereich (5) des Implantats (1) dient.

6. Implantat (1) nach einem der Ansprüche 1 bis 5,
wobei das Implantat (1) und/oder eine dem Implantat (1) zugeordnete Widerlager-Struktur (7) ausgebildet sind/ist, zumindest im implantierten Zustand antimikrobielle, antithrombotische oder zellwachstumshemmende Wirkstoffe freizusetzen.

7. Implantat (1) nach Anspruch 6,
wobei zum Freisetzen der Wirkstoffe die Fangeinrichtung (2) und/oder eine gegebenenfalls zum Implantat (1) gehörende Ersatzherzklappe (10) und/oder eine dem Implantat (1) zugeordnete Widerlager-Struktur (7) eine Beschichtung mit Wirkstoffen aufweist.

8. Implantat (1) nach Anspruch 6 oder 7,
wobei zum Freisetzen der Wirkstoffe das Implantat (1) oder Bestandteile des Implantats (1) und/oder eine dem Implantat (1) zugeordnete Widerlager-Struktur (7) mindestens eine bioresorbierbare Struktur aufweist, welche insbesondere eine Faden-, Vlies- und/oder Membranstruktur darstellt; und/oder
wobei das das Implantat (1) oder Bestandteile des Implantats (1) und/oder eine dem Implantat (1) zugeordnete Widerlager-Struktur (7) mindestens einen wirkstofffreisetzenden Bereich aufweisen/aufweist, der zumindest bereichsweise mit mindestens einem Polymermaterial beschichtet oder überzogen und ausgebildet ist, die Richtung einer Wirkstofffreisetzung zu steuern und/oder eine Wirkstofffreisetzung in Richtung des zur Beschichtung verwendeten mindestens einen Polymermaterials zumindest stark zu vermindern.

9. System zur Behandlung oder zum Ersatz einer an einer entzündeten, thrombosierten oder degenerierten Herzklappe (100) mit einem Implantat (1) nach einem der Ansprüche 1 bis 8 und einem Katheter (20) zum Einführen des Implantates (1) in den Körper eines Patienten, wobei das Implantat (1) ausgebildet ist, insbesondere in einem in Längsrichtung des Implantates (1) gestreckten und im Hinblick auf die Radialrichtung des Implantates (1) reduzierten Zustand in der Katheterspitze aufnehmbar zu sein, und wobei die Katheterspitze ausgebildet ist, das Implantat (1) insbesondere in seinem in Längsrichtung des Implantates (1) gestreckten und im Hinblick auf die Radialrichtung des Implantates (1) reduzierten Zustand aufzunehmen.

10. System nach Anspruch 9,
wobei das System ein zusätzliches und/oder unabhängiges insbesondere zumindest im Wesentlichen ringförmiges, selbstexpandierendes Implantat (7) aufweist, welches ausgebildet ist, insbesondere als Widerlager für das Implantat (1) zu dienen, und welches insbesondere über eine Streckung in einer radialen Richtung faltbar und in gefalteter Form in einer Katheterspitze aufnehmbar und derart am Implantationsort implantierbar ist, dass im implantierten Zustand des Implantates zwischen dem Implantat (1) und dem zusätzlichen und/oder unabhängigen Implantat (7) zumindest bereichsweise mindestens ein Klappensegel (103) der zu behandelnden Herzklappe (100) aufnehmbar ist.

11. System nach Anspruch 10,
wobei das zusätzliche und/oder unabhängige Implantat (7) ausgebildet ist, im komprimierten Zustand in der Katheterspitze so aufnehmbar zu sein, dass das zusätzliche und/oder unabhängige Implantat (7) im komprimierten Zustand nicht koaxial, sondern orthogonal zur Katheterspitzenachse ausgerichtet ist und mittels einer oder mehrerer druckstabiler Verbindungen auch schrittweise am Implantationsort freisetzbar ist.

## Claims

1. An implant (1) for treating and/or for replacing a diseased inflamed and/or infected heart valve (100), wherein the implant (1) comprises a capture device (2) able to be introduced into the body of a patient in a compressed state in minimally invasive manner and expanded at the site of implantation on the diseased heart valve (100), wherein the capture device (2) is designed to compartmentalize tissue changes or tissue deposits (101) in the form of heart valve vegetation or deposits at least when in the implanted and expanded state by way of the capture device (2) at least partially grasping and/or capturing the tissue change or deposit (101),
wherein the capture device (2) has an anchoring structure (3) for fixing and/or positioning the capture device (2) at the implantation site, and wherein the capture device (2) has a capturing structure (4) connected to the anchoring structure (3) which extends downstream from the anchoring structure (3), as viewed in the direction of blood flow, into a vessel (102) connected in terms of flow to the diseased heart valve (100) and comprises at least one clamping area (5) in the expanded state which is designed to interact with at least one heart valve leaflet (103) of the diseased heart valve (100) in the expanded and implanted state of the capture device (2) such that at least one heart valve leaflet (103) is pressed toward a vascular wall and at least partially enclosed by the capture device (2).

2. The implant (1) according to claim 1,
wherein the at least one clamping area (5) comprises at least one clamping arm or clamping bracket designed to at least partly expand radially upon the expansion of the capture device (2) in the implantation site, wherein the clamping area (5) is preferably designed to interact at a distal end region opposite from the anchoring structure (3) with a further abutment structure (7) to be implanted.

3. The implant (1) according to claim 1 or 2,
wherein the implant (1) further comprises an expandable replacement heart valve (10) able to in particular be introduced minimally invasively into the body of the patient in a compressed state and expanded at the site of implantation on the diseased heart valve (100) such that the replacement heart valve (10) assumes the function of a native heart valve (100), wherein the replacement heart valve (10) and the capture device (2) are preferably connected or connectable to one another, preferably by means of a common anchoring structure (3) which is designed to be positioned and fixed particularly in the area of the roots of the heart valve leaflets (103) of the native heart valve (100).

4. The implant (1) according to claim 3,
wherein the implant (1) is designed to gradually expand incrementally over time in the implantation site on the diseased native heart valve (100), wherein the capture device (2) expands in a first step and the replacement heart valve (10) expands in a second step; or
wherein the implant (1) is designed to gradually expand incrementally over time in the implantation site on the diseased native heart valve (100), wherein the replacement heart valve (10) expands in a first step and the capture device (2) expands in a second step.

5. The implant (1) according to one of claims 1 to 4,
wherein the implant (1) further comprises a structure (7) able to in particular be implanted separately from the capture device (2) and the heart valve (100) and which in particular exhibits a substantially annular self-expanding structure and is insertable into the pockets of the native heart valve (100) and serves as an abutment particularly for the capture device (2) or the clamping area (5) of the implant (1).

6. The implant (1) according to one of claims 1 to 5,
wherein the implant (1) and/or an abutment structure (7) associated with the implant (1) is/are designed to release antimicrobial, antithrombotic or cell growth-inhibiting active substances at least in the implanted state.

7. The implant (1) according to claim 6,
wherein for the release of the active substances, the capture device (2) and/or a replacement heart valve (10) which may be part of the implant (1) and/or an abutment structure (7) associated with the implant (1) has a coating of active substances.

8. The implant (1) according to claim 6 or 7,
wherein for the release of the active substances, the implant (1) or components of the implant (1) and/or an abutment structure (7) associated with the implant (1) have at least one bioresorbable structure which in particular constitutes a fibrous, non-woven and/or membrane structure; and/or
wherein the implant (1) or components of the implant (1) and/or an abutment structure (7) associated with the implant (1) have at least one active substance-releasing area which is at least partially coated or covered by at least one polymer material and designed to control the direction of active substance release and/or at least greatly reduce a release of active substance in the direction of the at least one polymer material used for the coating.

9. A system for treating or for replacing an inflamed, thrombosed or degenerated heart valve (100) comprising an implant (1) according to one of claims 1 to 8 and a catheter (20) for introducing the implant (1) into the body of a patient, wherein the implant (1) is designed to be able to be accommodated in the catheter tip particularly in a state elongated in the longitudinal direction of the implant (1) and reduced in relation to the radial direction of the implant (1), and wherein the catheter tip is designed to accommodate the implant (1) particularly in its state elongated in the longitudinal direction of the implant (1) and reduced in relation to the radial direction of the implant (1).

10. The system according to claim 9,
wherein the system has an additional and/or independent particularly at least substantially annular self-expanding implant (7) which is designed in particular to serve as an abutment for the implant (1) and in particular able to be folded by radial elongation and accommodated in folded form in a catheter tip and implanted at the implantation site such that in the implanted state of the implant (1), at least some areas of at least one valve leaflet (103) of the heart valve (100) to be treated can be accommodated between the implant (1) and the additional and/or independent implant (7).

11. The system according to claim 10,
wherein the additional and/or independent implant (7) is designed to be able to be accommodated in the catheter tip in compressed state such that the additional and/or independent implant (7) is not aligned coaxially but rather orthogonally to the catheter tip axis in the compressed state and is also able to be incrementally released at the implantation site by means of one or more pressure-stable connections.

## Revendications

1. Implant (1) pour le traitement et/ou le remplacement d'une valve cardiaque (100) atteinte d'une inflammation et/ou d'une infection,
dans lequel
l'implant (1) présente un dispositif de capture (2) qui, à l'état comprimé, peut être introduit par voie mini-invasive dans le corps du patient et peut être expansé sur le site d'implantation au niveau de la valve cardiaque atteinte (100),
le dispositif de capture (2) est réalisé pour, au moins à l'état implanté et expansé, compartimenter des modifications tissulaires ou des dépôts tissulaires (101) sous forme de végétations ou dépôts de la valve cardiaque, du fait que la modification ou le dépôt tissulaire (101) est entouré(e) et/ou reçu(e) au moins localement par le dispositif de capture (2),
le dispositif de capture (2) présente une structure d'ancrage (3) pour fixer et/ou positionner le dispositif de capture (2) sur le site d'implantation, et
le dispositif de capture (2) présente une structure de capture (4) qui est reliée à la structure d'ancrage (3) et qui, vue dans le sens du flux sanguin, s'étend de la structure d'ancrage (3) en aval jusque dans un vaisseau (102) en communication fluidique avec la valve cardiaque atteinte (100), et qui présente, à l'état expansé, au moins une zone de serrage (5) qui est réalisée pour, à l'état expansé et implanté du dispositif de capture (2), interagir avec au moins un feuillet valvulaire (103) de la valve cardiaque atteinte (100) de telle sorte qu'au moins un feuillet valvulaire (103) est pressé en direction d'une paroi vasculaire et est au moins partiellement entouré par le dispositif de capture (2).

2. Implant (1) selon la revendication 1,
dans lequel ladite au moins une zone de serrage (5) comprend au moins un bras de serrage ou un étrier de serrage qui est réalisé pour être expansé au moins localement dans la direction radiale lors de l'expansion du dispositif de capture (2) sur le site d'implantation,
la zone de serrage (5) est de préférence réalisée pour interagir avec une autre structure de contrebutée à implanter (7), dans une zone d'extrémité distale détournée de la structure d'ancrage (3).

3. Implant (1) selon la revendication 1 ou 2,
dans lequel l'implant (1) comprend en outre une valve cardiaque de remplacement (10) expansible qui, à l'état comprimé, peut être introduite en particulier par voie mini-invasive dans le corps du patient et qui peut être expansée sur le site d'implantation au niveau de la valve cardiaque atteinte (100), de telle sorte que la valve cardiaque de remplacement (10) assume la fonction d'une valve cardiaque native (100), la valve cardiaque de remplacement (10) et le dispositif de capture (2) étant de préférence reliés ou pouvant être reliés l'un à l'autre, de préférence par l'intermédiaire d'une structure d'ancrage commune (3) qui est réalisée pour être positionnée et fixée en particulier dans la zone des racines des feuillets valvulaires (103) de la valve cardiaque native (100).

4. Implant (1) selon la revendication 3,
dans lequel l'implant (1) est réalisé pour être expansé progressivement et successivement dans le temps sur le site d'implantation au niveau de la valve cardiaque native atteinte (100), le dispositif de capture (2) étant expansé dans une première étape, et la valve cardiaque de remplacement (10) étant expansée dans une deuxième étape ; ou
l'implant (1) est réalisé pour être expansé progressivement et successivement dans le temps sur le site d'implantation au niveau de la valve cardiaque native atteinte (100), la valve cardiaque de remplacement (10) étant expansée dans une première étape, et le dispositif de capture (2) étant expansé dans une deuxième étape.

5. Implant (1) selon l'une des revendications 1 à 4,
dans lequel l'implant (1) présente en outre une structure (7) implantable en particulier séparément du dispositif de capture (2) et de la valve cardiaque (100), qui présente en particulier une conception auto-expansible de forme sensiblement annulaire et qui peut être insérée dans les valvules de la valve cardiaque native (100) et qui sert de contrebutée en particulier pour le dispositif de capture (2) ou la zone de serrage (5) de l'implant (1).

6. Implant (1) selon l'une des revendications 1 à 5,
dans lequel l'implant (1) et/ou une structure de contrebutée (7) associée à l'implant (1) sont/est réalisé(e)(s) pour libérer, au moins à l'état implanté, des principes actifs antimicrobiens, antithrombotiques ou inhibiteurs de la croissance cellulaire.

7. Implant (1) selon la revendication 6,
dans lequel, pour libérer les principes actifs, le dispositif de capture (2) et/ou une valve cardiaque de remplacement (10) appartenant le cas échéant à l'implant (1) et/ou une structure de contrebutée (7) associée à l'implant (1) présente un revêtement avec des principes actifs.

8. Implant (1) selon la revendication 6 ou 7,
dans lequel, pour libérer les principes actifs, l'implant (1) ou des composants de l'implant (1) et/ou une structure de contrebutée (7) associée à l'implant (1) présente(nt) au moins une structure bio-résorbable qui représente en particulier une structure de fil, de nappe et/ou de membrane ; et/ou
l'implant (1) ou des composants de l'implant (1) et/ou une structure de contrebutée (7) associée à l'implant (1) présente(nt) au moins une zone libérant un principe actif, qui est revêtue ou recouverte au moins localement d'au moins un matériau polymère et qui est réalisée pour contrôler la direction d'une libération de principe actif et/ou pour réduire au moins fortement une libération de principe actif dans la direction dudit au moins un matériau polymère utilisé pour le revêtement.

9. Système pour le traitement ou le remplacement d'une valve cardiaque (100) enflammée, thrombosée ou dégénérée, comprenant un implant (1) selon l'une des revendications 1 à 8 et un cathéter (20) pour introduire un implant (1) dans le corps d'un patient,
dans lequel l'implant (1) est réalisé pour pouvoir être reçu dans la pointe du cathéter, en particulier dans un état étiré dans la direction longitudinale de l'implant (1) et réduit par rapport à la direction radiale de l'implant (1), et la pointe du cathéter est réalisée pour recevoir l'implant (1), en particulier dans son état étiré dans la direction longitudinale de l'implant (1) et réduit par rapport à la direction radiale de l'implant (1).

10. Système selon la revendication 9,
dans lequel le système comprend un implant auto-expansible (7) supplémentaire et/ou indépendant, en particulier de forme au moins sensiblement annulaire, qui est réalisé pour servir en particulier de contrebutée à l'implant (1) et qui est en particulier pliable par un étirement dans une direction radiale et peut être reçu sous forme pliée dans une pointe de cathéter et peut être implanté sur le site d'implantation de telle sorte qu'à l'état implanté de l'implant, au moins un feuillet valvulaire (103) de la valve cardiaque (100) à traiter peut être reçu au moins localement entre l'implant (1) et l'implant (7) supplémentaire et/ou indépendant.

11. Système selon la revendication 10,
dans lequel l'implant supplémentaire et/ou indépendant (7) est réalisé pour pouvoir être reçu, à l'état comprimé, dans la pointe du cathéter de telle sorte que l'implant (7) supplémentaire et/ou indépendant, à l'état comprimé, n'est pas orienté coaxialement mais orthogonalement par rapport à l'axe de la pointe du cathéter et peut également être libéré progressivement sur le site d'implantation au moyen d'une ou plusieurs liaisons stables à la pression.
